# EUROPEAN PATENT APPLICATION

(11) **EP 4 817 436 A1**
(43) Date of publication of application: **07.10.2026**
(21) Application number: 24896437.1
(22) Date of filing: 22.11.2024
(51) Int. Cl.: A24F 40/40, A24F 40/42, A24F 40/10, A61M 11/00

(54) **ATOMIZER AND ELECTRONIC ATOMIZATION DEVICE**

(30) Priority: 30.11.2023 CN 202323273995 U
(71) Applicant: Shenzhen First Union Technology Co., Ltd., Shenzhen, Guangdong 518000 (CN)
(72) Inventor: LIANG, Xiubi, Shenzhen, Guangdong 518000 (CN); LU, Linhai, Shenzhen, Guangdong 518000 (CN); XIA, Zhicong, Shenzhen, Guangdong 518000 (CN); XU, Zhongli, Shenzhen, Guangdong 518000 (CN); LI, Yonghai, Shenzhen, Guangdong 518000 (CN)
(74) Representative: Proi World Intellectual Property GmbH
(86) International application number: PCT/CN2024/133860
(87) International publication number: WO 2025/113346

(57) **Abstract**

The present application discloses an atomizer and an electronic atomization device. The atomizer comprises: a housing; a retaining seat arranged in the housing and cooperating with the housing to form a liquid storage cavity, wherein the retaining seat comprises a top wall and a side wall which define an accommodating cavity, the top wall is provided with ventilation holes, the side wall comprises a first side wall and a second side wall arranged opposite to each other, the first side wall is provided with a liquid inlet, and the second side wall is provided with an assembly hole; a base at least partially arranged in the housing and provided with ventilation columns, wherein the ventilation columns penetrate through the ventilation holes and cooperate with the inner surfaces of the ventilation holes to form ventilation channels, one end of each ventilation channel is communicated with the liquid storage cavity, and the other end of the ventilation channel is communicated with the atmosphere; and an atomization core arranged in the accommodating cavity, wherein the atomization core is installed into the accommodating cavity by means of the assembly hole. By means of the arrangement, during ventilation, bubbles are not prone to attaching to a liquid suctioning surface of the atomization core, ensuring smooth liquid flow, and making ventilation more stable; and the atomization core is installed into the accommodating cavity by means of the assembly hole, so that an assembly method is simpler and efficiency is higher.

## Description

### CROSS-REFERENCE TO RELATED APPLICATIONS

This application claims priority to the Chinese Patent Application No. 202323273995.2, filed with China National Intellectual Property Administration on November 30, 2023 and entitled "ATOMIZER AND ELECTRONIC ATOMIZATION DEVICE", which is incorporated herein by reference in its entirety.

### TECHNICAL FIELD

This application relates to the field of atomization technology, and in particular to an atomizer and an electronic atomization device.

### BACKGROUND

An electronic atomization device generally includes an atomizer and a power supply assembly, and the power supply assembly is electrically connected to the atomizer for suppling power to the atomizer. The atomizer usually includes an atomizable liquid substrate and an atomization core. The atomization core includes a porous substrate and a heating element. The porous substrate has a large number of micro pores inside for absorbing the liquid substrate, and the heating element heats and atomizes the liquid substrate to generate an aerosol. As a user inhales, the liquid substrate in a liquid storage cavity is continuously consumed, and air needs to enter the liquid storage cavity from the outside to maintain the air pressure balance inside the liquid storage cavity, preventing excessive negative pressure in the liquid storage cavity from causing the liquid substrate to stop being supplied. Therefore, it is necessary to provide a ventilation channel in the atomizer so that air enters the liquid storage cavity through a ventilation channel.

Currently, for the atomizer that atomizes laterally, when the user inhales, external air enters the liquid storage cavity through the ventilation channel, bubbles are prone to be generated, and the bubbles tend to attach to the surface of the porous substrate, affecting the liquid absorption of the porous substrate, easily leading to poor liquid flow. As a result, dry heating of the atomization core is easily caused, and unstable ventilation tends to occur.

### SUMMARY

This application mainly provides an atomizer and an electronic atomization device, to resolve a problem in the related art that in the ventilation process of the atomizer, bubbles tend to attach to a surface of a porous substrate, resulting in poor liquid flow and unstable ventilation.

To resolve the foregoing technical problem, a technical solution adopted by this application is to provide an atomizer, including:
a housing; a retaining seat arranged in the housing and cooperating with the housing to form a liquid storage cavity for storing a liquid substrate, where the retaining seat includes a top wall and a side wall connected to each other; the top wall and the side wall enclose an accommodating cavity, and the top wall is provided with a ventilation hole, where the side wall includes a first side wall and a second side wall arranged opposite to each other, the first side wall is provided with a liquid inlet, the liquid inlet is in communication with the liquid storage cavity, and the second side wall is provided with an assembly hole. A base is at least partially arranged in the housing and located at an end of the retaining seat away from the liquid storage cavity, where a ventilation column is arranged on the base, the ventilation column penetrates the ventilation hole, and the ventilation column cooperates with an inner surface of the ventilation hole to form a ventilation channel, one end of the ventilation channel is in communication with the liquid storage cavity, and the other end of the ventilation channel is in communication with an external atmosphere. An atomization core is arranged in the accommodating cavity, where the atomization core includes a liquid absorbing surface for receiving the liquid substrate, the liquid absorbing surface is communication with the liquid storage cavity through the liquid inlet, where the assembly hole is configured to allow the atomization core to be mounted in the accommodating cavity through the assembly hole.

In some implementations, the ventilation column is located on a side of the atomization core away from the liquid inlet and is spaced apart from the atomization core.

In some implementations, an end portion of the side wall away from the liquid storage cavity is provided with an annular groove, and a bottom wall of the annular groove is provided with two arc-shaped grooves spaced apart from each other. The base includes a mounting portion and an annular flange, and the annular flange is connected to a side of the mounting portion close to the retaining seat; two support portions spaced apart from each other are arranged on the annular flange in a direction away from the mounting portion; and the annular flange is embedded in the annular groove, and the two support portions are embedded in the two arc-shaped grooves in a one-to-one correspondence.

In some implementations, the side wall further includes a third side wall and a fourth side wall connected between the first side wall and the second side wall, the first side wall and the second side wall are arranged opposite to each other in a thickness direction of the atomizer, and the third side wall and the fourth side wall are arranged opposite to each other in a width direction of the atomizer. The two arc-shaped grooves are provided on the third side wall and the fourth side wall in a one-to-one correspondence.

In some implementations, one end of the ventilation column is connected to the base, the ventilation column passes through the accommodating cavity, and the other end of the ventilation column extends into the liquid storage cavity, for the ventilation channel to come into communication with the accommodating cavity.

In some implementations, a side surface of the ventilation column is provided with a ventilation groove, one end of the ventilation groove extends to an end surface of an end of the ventilation column away from the base, and the other end of the ventilation groove is in communication with the accommodating cavity.

In some implementations, the atomizer further includes a fitting member, and the fitting member is mounted in the assembly hole; and/or the atomization core further includes an atomization surface, the atomization surface is arranged toward the assembly hole, the atomization surface cooperates with the accommodating cavity to form an atomization cavity; an air inlet cavity is defined between the base and the retaining seat, and the air inlet cavity is in communication with the atomization cavity; the base is provided with an air inlet column and two electrode mounting holes, and the two electrode mounting holes are respectively arranged on two sides of the air inlet column; and one end of the air inlet column is in communication with the air inlet cavity, and the other end of the air inlet column is in communication with the external atmosphere.

In some implementations, the atomizer further includes two conductive electrodes, and the conductive electrodes correspondingly penetrate the electrode mounting holes; the conductive electrodes are configured to be inserted from a bottom of the retaining seat into the atomization cavity and to abut against the atomization surface; and the ventilation column is configured to be inserted from the bottom of the retaining seat into the ventilation holes.

In some implementations, the housing is provided with an air outlet tube inside, and the top wall of the retaining seat is provided with an air outlet hole, one end of the air outlet hole in communication with the atomization cavity, and the other end of the air outlet hole in communication with the air outlet tube; and the air inlet column, the atomization cavity, and the air outlet tube are coaxially arranged.

To resolve the foregoing technical problem, another technical solution adopted by this application is to provide an electronic atomization device, including: an atomizer, which is any one of the atomizers described above; and a power supply assembly electrically connected to the atomizer, configured to supply energy to the atomizer.

The beneficial effects pf this application are as follows: different from the situation in the related art, this application discloses an atomizer and an electronic atomization device. The atomizer includes: a housing; a retaining seat arranged in the housing and cooperating with the housing to form a liquid storage cavity for storing a liquid substrate, where the retaining seat includes a top wall and a side wall connected to each other; the top wall and the side wall enclose an accommodating cavity, and the top wall is provided with a ventilation hole, where the side wall includes a first side wall and a second side wall arranged opposite to each other, the first side wall is provided with a liquid inlet, the liquid inlet is in communication with the liquid storage cavity, and the second side wall is provided with an assembly hole; a base, at least partially arranged in the housing and located at an end of the retaining seat away from the liquid storage cavity, where a ventilation column is arranged on the base, the ventilation column penetrates the ventilation hole; and the ventilation column cooperates with an inner surface of the ventilation hole to form a ventilation channel, one end of the ventilation channel is in communication with the liquid storage cavity, and the other end of the ventilation channel is in communication with an external atmosphere; an atomization core arranged in the accommodating cavity, where the atomization core includes a liquid absorbing surface for receiving the liquid substrate, the liquid absorbing surface is in communication with the liquid storage cavity through the liquid inlet, where the assembly hole is configured to allow the atomization core to be mounted in the accommodating cavity through the assembly hole. Through the arrangement, the ventilation column is arranged on the base, the top wall of the retaining seat is provided with a ventilation hole, and the ventilation column cooperates with an inner surface of the ventilation hole to form a ventilation channel, which can ventilate the liquid storage cavity. In addition, an outlet of the ventilation channel is located on the top wall of the retaining seat. During ventilation, bubbles are not prone to attach to a liquid absorbing surface of the atomization core, ensuring smooth liquid flow, and making ventilation more stable. And the atomization core is mounted in the accommodating cavity through the assembly hole on the second side wall of the retaining seat, so that the assembly method is simpler and efficiency is higher.

### BRIEF DESCRIPTION OF THE DRAWINGS

To describe the technical solutions in embodiments of this application or in the related art more clearly, the accompanying drawings required for describing the embodiments or the related art are to be briefly described below. Apparently, the accompanying drawings in the following description show only some embodiments of this application, and a person of ordinary skill in the art may still derive other accompanying drawings from the accompanying drawings without creative efforts.
FIG. 1 is a schematic structural diagram of an embodiment of an electronic atomization device according to this application;
FIG. 2 is a schematic structural diagram of an atomizer of the electronic atomization device provided in FIG. 1;
FIG. 3 is a schematic cross-sectional view of the atomizer provided in FIG. 2;
FIG. 4 is another schematic cross-sectional view of the atomizer provided in FIG. 2;
FIG. 5 is a schematic top structural diagram of a retaining seat of the atomizer provided in FIG. 3;
FIG. 6 is a schematic bottom structural diagram of the retaining seat provided in FIG. 5;
FIG. 7 is a schematic cross-sectional view of the retaining seat provided in FIG. 5;
FIG. 8 is another schematic cross-sectional view of the retaining seat provided in FIG. 5;
FIG. 9 is a schematic structural diagram of a base of the atomizer provided in FIG. 3;
FIG. 10 is a schematic cross-sectional view of the base provided in FIG. 9; and
FIG. 11 is a schematic structural diagram of an atomization core of the atomizer provided in FIG. 3.

### DETAILED DESCRIPTION

The technical solutions in embodiments of this application are clearly and completely described in the following with reference to the accompanying drawings in the embodiments of this application. Apparently, the described embodiments are merely some rather than all of the embodiments of this application. Based on the embodiments of this application, all other embodiments obtained by a person of ordinary skill in the art without making creative efforts fall within the scope of protection of this application.

The terms "first", "second", and "third" in the embodiments of this application are merely for a purpose of description, and shall not be understood as an indication or implication of relative importance or implicit indication of the quantity of indicated technical features. Therefore, a feature defined with "first", "second", and "third" can explicitly or implicitly includes at least one of the features. In descriptions of this application, unless otherwise stated, "plurality of" means two or more, such as two or three. In addition, the terms "include", "have", and any variant thereof are intended to cover non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device.

"Embodiment" mentioned in this specification means that specific features, structures, or characteristics described with reference to the embodiments may be included in at least one embodiment of this application. The term appearing at different positions of this specification may not refer to the same embodiment or an independent or alternative embodiment that is mutually exclusive with other embodiments. A person skilled in the art explicitly or implicitly understands that embodiments described in this specification may be combined with other embodiments.

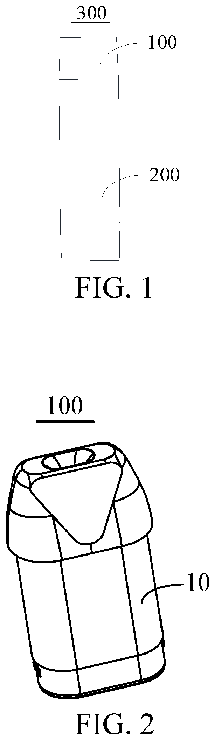

Refer to FIG. 1. FIG. 1 is a schematic structural diagram of an embodiment of an electronic atomization device according to this application.

Refer to FIG. 1. This application provides an electronic atomization device 300. The electronic atomization device 300 may be used for atomizing a liquid substrate. The electronic atomization device 300 includes an atomizer 100 and a power supply assembly 200 that are electrically connected to each other.

The atomizer 100 is configured to store the liquid substrate and atomize the liquid substrate to form an aerosol that can be inhaled by a user. The atomizer 100 may be specifically used in different fields, such as medical care, cosmetology, and leisure inhalation. In a specific embodiment, the atomizer 100 may be used in an electronic aerosol atomization device, to atomize a liquid substrate and generate an aerosol for inhalation by an inhaler. The following embodiments are described by using this leisure inhalation as an example.

For specific structures and functions of the atomizer 100, reference may be made to the specific structures and functions of the atomizer 100 involved in the following embodiments, same or similar technical effects may also be achieved, and details are not described herein.

A battery core (not shown in the figure), an electric connection terminal (not shown in the figure), a barometric pressure sensor (not shown in the figure), and a controller (not shown in the figure) are arranged in the power supply assembly 220. The electric connection terminal may be electrically connected to a conductive electrode 50 of the atomizer 100. When the user inhales using the electronic atomization device 300, the barometric pressure sensor senses a change in internal air pressure, and sends a sensing signal to the controller. The controller controls the battery core to supply electric energy to the atomizer 100 through the electric connection terminal. The atomization core 30 in the atomizer 100 then receives the electric energy and starts to heat and atomize the liquid substrate to generate an aerosol that can be used by the user. The power supply assembly 200 may further include other components such as a battery holder (not shown in the figure).

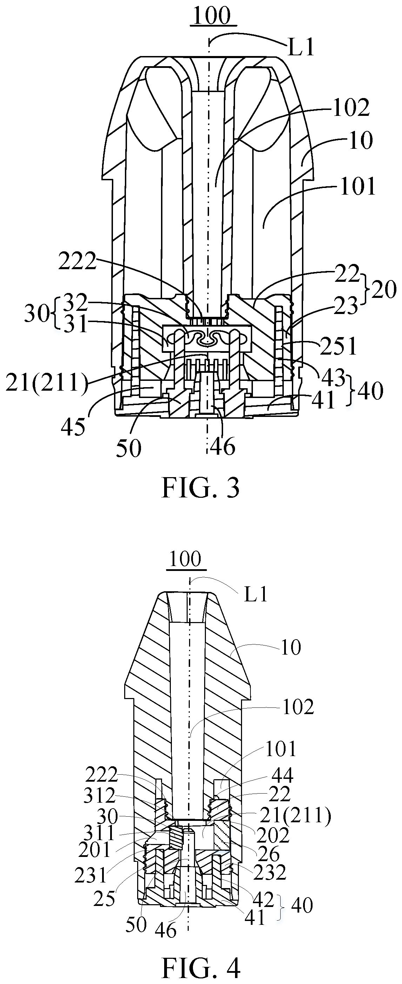

Refer to FIG. 2 to FIG. 4. FIG. 2 is a schematic structural diagram of an atomizer of the electronic atomization device provided in FIG. 1; FIG. 3 is a schematic cross-sectional view of the atomizer provided in FIG. 2; and FIG. 4 is another schematic cross-sectional view of the atomizer provided in FIG. 2.

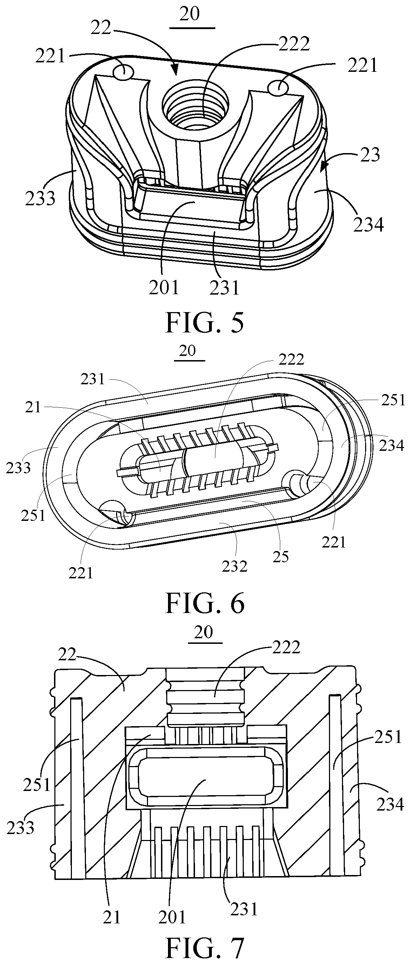

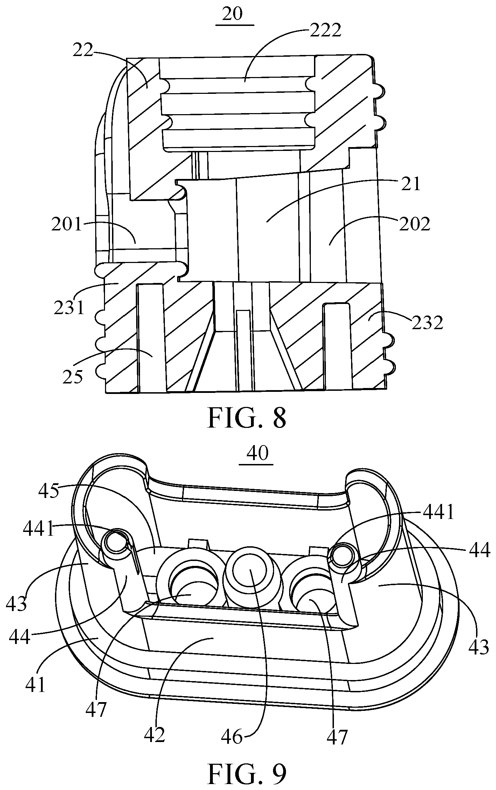

Refer to FIG. 2 to FIG. 4. The atomizer 100 includes a housing 10, a retaining seat 20, an atomization core 30, and a base 40. The retaining seat 20 is arranged in the housing 10, and cooperates with the housing 10 to form a liquid storage cavity 101. The liquid storage cavity 101 is configured to store a liquid substrate. The retaining seat 20 includes a top wall 22 and a side wall 23 connected to each other. The side wall 23 includes a first side wall 231 and a second side wall 232 arranged opposite to each other. The first side wall 231 is provided with a liquid inlet 201. The liquid inlet 201 is in flow communication with the liquid storage cavity 101. The second side wall 232 is provided with an assembly hole 202. The base 40 is at least partially arranged in the housing 10, and located at an end of the retaining seat 20 away from the liquid storage cavity 101. The top wall 22 of the retaining seat 20 is provided with a ventilation hole 221 (referring to FIG. 5). A ventilation column 44 is arranged on the base 40 (referring to FIG. 9). The ventilation column 44 penetrates the ventilation hole 221. The ventilation column 44 cooperates with an inner surface of the ventilation hole 221 to form a ventilation channel. One end of the ventilation channel is in communication with the liquid storage cavity 101, and the other end of the ventilation channel is in communication with an external atmosphere. The top wall 22 and the side wall 23 of the retaining seat 20 enclose the accommodating cavity 21, and the atomization core 30 is arranged in the accommodating cavity 21. Specifically, the atomization core 30 includes a liquid absorbing surface 311, the liquid absorbing surface 311 is configured to receive the liquid substrate, and the liquid absorbing surface 311 is in communication with the liquid storage cavity 101 through the liquid inlet 201. In a specific implementation, the liquid absorbing surface 311 is arranged toward the liquid inlet 201. The assembly hole 202 is configured to allow the atomization core 30 to be mounted in the accommodating cavity 21 through the assembly hole 202.

It may be understood that, the ventilation hole 221 is provided on the top wall 22 of the retaining seat 20, and the ventilation column 44 is arranged on the base 40, the ventilation column 44 penetrates the ventilation hole 221, the ventilation column 44 cooperates with the inner surface of the ventilation hole 221 to form the ventilation channel, one end of the ventilation channel is in communication with the liquid storage cavity 101, and the other end of the ventilation channel is directly or indirectly in communication with the external atmosphere, so that the ventilation channel can ventilate the liquid storage cavity 101, balancing air pressure inside the liquid storage cavity 101, and avoiding dry heating of the atomization core 30 due to poor liquid flow caused by an imbalance of the air pressure inside the liquid storage cavity 101. In addition, an outlet of the ventilation channel is located on the top wall 22 of the retaining seat 20. During ventilation, bubbles are exported toward a direction away from the liquid absorbing surface 311 of the atomization core 30, and are not prone to attach to the liquid absorbing surface 311 of the atomization core 30, ensuring smooth liquid flow, further preventing dry heating of the atomization core 30, and making ventilation more stable. In addition, the assembly hole 202 is provided on the second side wall 232 of the retaining seat 20, and the atomization core 30 is mounted in the accommodating cavity 21 through the assembly hole 202 on the second side wall 232 of the retaining seat 20, so that the assembly can be simpler and assembly efficiency can be higher.

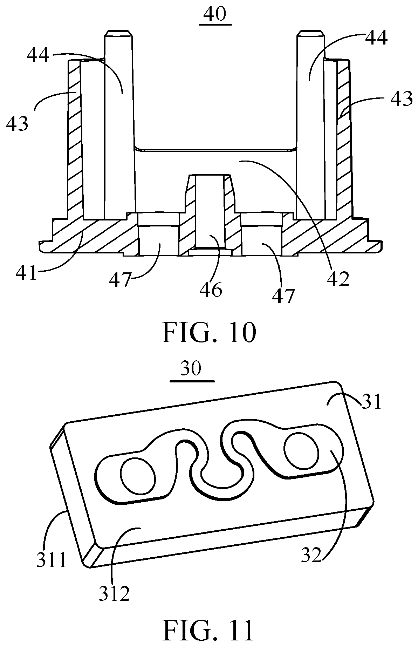

Refer to FIG. 5 to FIG. 11. FIG. 5 is a schematic top structural diagram of a retaining seat of the atomizer provided in FIG. 3; FIG. 6 is a schematic bottom structural diagram of the retaining seat provided in FIG. 5; FIG. 7 is a schematic cross-sectional view of the retaining seat provided in FIG. 5; FIG. 8 is another schematic cross-sectional view of the retaining seat provided in FIG. 5; FIG. 9 is a schematic structural diagram of a base of the atomizer provided in FIG. 3; FIG. 10 is a schematic cross-sectional view of the base provided in FIG. 9; and FIG. 11 is a schematic structural diagram of an atomization core of the atomizer provided in FIG. 3.

Specifically, in some implementations, there are two ventilation holes 221. The two ventilation holes 221 are spaced apart from each other on the top wall 22 of the retaining seat 20. As shown in FIG. 5 to FIG. 8, in a width direction of the atomizer 100, the two ventilation holes 221 are respectively located on two sides of the assembly hole 202. Refer to FIG. 2 to FIG. 4, FIG. 9, and FIG. 10. There are also two ventilation columns 44 on the base 40. The two ventilation columns 44 penetrate two ventilation holes 221 in a one-to-one correspondence, forming two ventilation channels. It may be understood that, by providing two ventilation holes 221 and two ventilation columns 44 to cooperate to form two ventilation channels, ventilation efficiency can be improved, and the air pressure inside the liquid storage cavity 101 is more efficiently balanced, thereby ensuring ventilation stability.

In other implementations, the number of the ventilation holes 221 and the number of the ventilation columns 44 may be correspondingly set to other numbers. For example, the number of the ventilation holes 221 and the number of the ventilation columns 44 are set to any other number, such as one, three, four, or five, to form any other number of ventilation channels for ventilating the liquid storage cavity 101.

In some implementations, the ventilation column 44 is located on a side of the atomization core 30 away from the liquid inlet 201, and is spaced apart from the atomization core 30. In a specific implementation, as shown in FIG. 4, there are two ventilation columns 44. The two ventilation columns 44 are located on the side of the atomization core 30 away from the liquid inlet 201 in a thickness direction of the atomizer 100, and are spaced apart from the atomization core 30. By providing the ventilation holes 221 and the ventilation columns 44 on the side of the atomization core 30 away from the liquid inlet 201, the ventilation channels are spaced apart from the liquid inlet 201 and the liquid absorbing surface 311 of the atomization core 30. A sufficient distance is provided between the ventilation columns 44 and the liquid inlet 201, so that bubbles generated during ventilation are less prone to contact the liquid absorbing surface 311 of the atomization core 30, and the bubbles do not attach to the liquid absorbing surface 311 of the atomization core 30 to affect a liquid absorbing effect of the liquid absorbing surface 311, thereby effectively preventing dry heating of the atomization core 30.

Refer to FIG. 5 to FIG. 10. In some implementations, an end portion of a side wall 23 of the retaining seat 20 away from the liquid storage cavity 101 is provided with an annular groove 25, and a bottom wall of the annular groove 25 is provided with two arc-shaped grooves 251 spaced apart from each other. The base 40 includes a mounting portion 41 and an annular flange 42. The annular flange 42 is connected to a side of the mounting portion 41 close to the retaining seat 20. Two support portions 43 spaced apart from each other are arranged on the annular flange 42 in a direction away from the mounting portion 41. The annular flange 42 is embedded in the annular groove 25, and the two support portions 43 are embedded in the two arc-shaped grooves 251 in a one-to-one correspondence, so as to achieve a connection between the retaining seat 20 and the base 40. The material of the base 40 may include a hard material such as plastic, while the retaining seat 20 is a flexible member. Specifically, the material of the retaining seat 20 is a flexible material. For example, the retaining seat 20 is made of a flexible adhesive material such as silicone, rubber, or thermoplastic elastomer (TPE). By embedding the annular flange 42 of the base 40 into the annular groove 25 of the retaining seat 20, the side wall 23 of the retaining seat 20 can seal between the base 40 and an inner surface of the housing 10, which helps prevent leakage of the liquid substrate in the liquid storage cavity 101. Furthermore, by connecting two support portions 43 on the annular flange 42 of the base 40, the support portions 43 being arc-shaped, providing two arc-shaped grooves 251 on the bottom wall of the annular groove 25 of the retaining seat 20, and embedding the two support portions 43 into the two arc-shaped grooves 251 in a one-to-one correspondence, it is more effective for the support portions 43 to support the side wall 23 of the retaining seat 20, further improving sealing reliability between the base 40 and the housing 10.

Specifically, in some implementations, the side wall 23 of the retaining seat 20 further includes a third side wall 233 and a fourth side wall 234, and the third side wall 233 and the fourth side wall 234 are connected between the first side wall 231 and the second side wall 232. In a specific implementation, the first side wall 231 and the second side wall 232 are arranged opposite to each other in the thickness direction of the atomizer 100, and the first side wall 231 and the second side wall 232 are rectangular. The third side wall 233 and the fourth side wall 234 are arranged opposite to each other in the width direction of the atomizer 100, and the third side wall 233 and the fourth side wall 234 are arc-shaped. The two arc-shaped grooves 251 are provided on the third side wall 233 and the fourth side wall 234 in a one-to-one correspondence. In other implementations, the first side wall 231 and the second side wall 232 may alternatively be arranged opposite to each other in another direction different from the thickness direction of the atomizer 100, the third side wall 233 and the fourth side wall 234 may alternatively be arranged opposite to each other in another direction different from the width direction of the atomizer 100, and the first side wall 231, the second side wall 232, the third side wall 233, and the fourth side wall 234 may alternatively be in any other shapes.

In some implementations, one end of the ventilation column 44 is connected to the base 40, the ventilation column 44 passes through the accommodating cavity 21, and the other end of the ventilation column 44 extends into the liquid storage cavity 101, for the ventilation channel to come into communication with the accommodating cavity 21. Specifically, the housing 10 defines a central axis L1 of the atomizer 100. Refer to FIG. 3 and FIG. 4. In some implementations, the liquid absorbing surface 311 of the atomization core 30 is arranged parallel to the central axis L1 of the atomizer 100. Refer to FIG. 9 and FIG. 10. In a specific implementation, the ventilation column 44 is located in the annular flange 42 of the base 40, and is arranged parallel to the central axis L1 of the atomizer 100. An end of the ventilation column 44 away from the liquid storage cavity 101 is connected to the mounting portion 41 of the base 40, and a side surface of the ventilation column 44 is connected to an inner side surface of the annular flange 42 and connected to the support portion 43. Specifically, the ventilation column 44 is configured to be inserted into the ventilation hole 221 from a bottom of the retaining seat 20, and an end of the ventilation column 44 away from the base 40 extends into the liquid storage cavity 101. That is, the end of the ventilation column 44 away from the base 40 protrudes from the top wall 22 of the retaining seat 20 and extends into the liquid storage cavity 101, and an end opening of an end of the ventilation channel close to the liquid storage cavity 101 is located in the liquid storage cavity 101.

It may be understood that, arranging the ventilation column 44 in a direction parallel to the central axis L1 of the atomizer 100 allows the ventilation hole 221 to be correspondingly arranged as a straight through hole, which is more conductive to improving ventilation stability. Moreover, the ventilation column 44 is located inside the annular flange 42, so that when the retaining seat 20 and the base 40 are fixedly connected, the ventilation column 44 is partially located in the accommodating cavity 21. The end of the ventilation channel formed by the cooperation of ventilation column 44 and the ventilation hole 221 away from the liquid storage cavity 101 is directly in communication with the accommodating cavity 21, so that after external gas enters the accommodating cavity 21, part of gas may directly enter the ventilation channels via the accommodating cavity 21, thereby achieving ventilation of the liquid storage cavity 101 and improving ventilation efficiency. In other implementations, the end of the ventilation channel away from the liquid storage cavity 101 may alternatively be directly in communication with the external atmosphere. In other implementations, the ventilation column 44 may alternatively be not arranged parallel to the central axis L1 of the atomizer 100. For example, the ventilation column 44 may be arranged obliquely to the central axis L1 of the atomizer 100, and the ventilation hole 221 may alternatively be an inclined through hole, provided that the ventilation column 44 and the ventilation hole 221 can cooperate to form the ventilation channel and ventilate the liquid storage cavity 101.

In a specific implementation, as shown in FIG. 9, the side surface of the ventilation column 44 is provided with a ventilation groove 441. One end of the ventilation groove 441 extends to an end surface of an end of the ventilation column 44 away from the base 40, and the other end of the ventilation groove is in communication with the accommodating cavity 21 of the retaining seat 20. The ventilation column 44 is embedded in the ventilation hole 221, and the ventilation groove 441 of the ventilation column 44 cooperates with the inner surface of the ventilation hole 221 to form the ventilation channel, to ventilate the liquid storage cavity 101. Optionally, the ventilation groove 441 has an appropriate width or depth to generate a capillary effect. The ventilation column 44 is made of a hard material such as plastic, which can keep an air inlet cross-sectional area of the ventilation channel substantially stable. Due to the capillary effect, the ventilation groove 441 can prevent, to some extent, the liquid substrate in the liquid storage cavity 101 from leaking through the ventilation groove 441, while a small amount of the liquid substrate from the liquid storage cavity 101 can be retained in the ventilation groove 441. Only when negative pressure in the liquid storage cavity 101 reaches a specific level, air in the accommodating cavity 21 may enter the liquid storage cavity 101 through the ventilation channel driven by a pressure difference between the inside and the outside, thereby reducing the negative pressure inside the liquid storage cavity 101. Moreover, an end opening of an end of the ventilation groove 441 close to the liquid storage cavity 101 extends to the end surface of the end of the ventilation column 44 away from the base 40, and the end opening of the end of the ventilation groove 441 close to the liquid storage cavity 101 is higher than the liquid absorbing surface 311. Ventilation bubbles caused by the external air replenishing and entering the liquid storage cavity 101 escape in a direction away from the liquid absorbing surface 311, preventing the bubbles that escape from the end opening of the end of the ventilation groove 441 close to the liquid storage cavity 101 from accumulating near the liquid absorbing surface 311 and hindering liquid absorbing.

Specifically, as shown in FIG. 9, the two ventilation columns 44 are each provided with a ventilation groove 441, forming two ventilation channels. In other implementations, ventilation column 44 may alternatively be each provided with any number of ventilation grooves 441, such as two or three, to form a plurality of ventilation channels and improve ventilation efficiency. A shape of the ventilation groove 441 may be a straight through groove, or may be a curved groove, provided that the liquid storage cavity 101 can be ventilated.

In some other implementations, the ventilation groove 441 may alternatively be provided on the inner surface of the ventilation hole 221. That is, the ventilation groove 441 is provided on a flexible material. One end of the ventilation groove 441 extends to communicate with the liquid storage cavity 101, and the other end of the ventilation groove 441 extends to communicate with the accommodating cavity 21. An outer side surface of the ventilation column 44 cooperates with the ventilation groove 441 on the inner surface of the ventilation hole 221 to form the ventilation channel, thereby achieving ventilation of the liquid storage cavity 101. Similarly, the inner surface of the ventilation hole 221 may be provided with one or more ventilation grooves 441, and the shape of the ventilation groove 441 may also be arbitrarily configured.

In some other implementations, the outer side surface of the ventilation column 44 and the inner surface of the ventilation holes 221 may alternatively be at least partially spaced apart. The ventilation channel is formed by a gap between the outer side surface of the ventilation column 44 and the inner surface of the ventilation hole 221. One end of the gap is in communication with the liquid storage cavity 101, and the other end is in communication with the external atmosphere, so as to ventilate the liquid storage cavity 101.

In some other implementations, the ventilation channel may alternatively include an air guiding through hole passing through a side wall of the accommodating cavity 21. One end of the air guiding through hole is in communication with the liquid storage cavity 101, and the other end of the air guiding through hole is in communication with the external atmosphere, allowing air to enter the liquid storage cavity 101. Optionally, the air guiding through hole may have a capillary structure.

In other implementations, the atomizer 100 may include a plurality of ventilation channels. The plurality of ventilation channels may include any one or more of the foregoing ventilation channels. For example, the atomizer 100 includes two ventilation channels, one of the ventilation channels is formed by the cooperation of the ventilation groove 441 on the outer side surface of the ventilation column 44 with the inner surface of the ventilation hole 221, and the other ventilation channel is formed by the cooperation of the ventilation groove 441 on the inner surface of the ventilation hole 221 with the outer side surface of the ventilation column 44, which may be designed as required. It may be understood that providing a plurality of ventilation channels can improve ventilation efficiency of the atomizer 100, and more effectively avoid poor liquid flow caused by an imbalance of the air pressure inside the liquid storage cavity 101.

Furthermore, refer to FIG. 3 and FIG. 4. The atomizer 100 further includes a fitting member 26. After the atomization core 30 is mounted in the accommodating cavity 21 through the assembly hole 202, the fitting member 26 is mounted in the assembly hole 202 to increase structural strength of the retaining seat 20, and prevent the retaining seat 20 from being greatly deformed when being mounted in the housing 10. Moreover, the fitting member 26 may further seal the assembly hole 202, to prevent the liquid substrate in the liquid storage cavity 101 from leaking into the accommodating cavity 21. The fitting member 26 and the assembly hole 202 may be assembled and connected by an interference fit, to better prevent the liquid substrate in the liquid storage cavity 101 from leaking from the assembly hole 202 into the accommodating cavity 21.

Refer to FIG. 3, FIG. 4, and FIG. 11. The atomization core 30 further includes an atomization surface 312. The atomization surface 312 is arranged opposite to the liquid absorbing surface 311. The atomization surface 312 is arranged toward the assembly hole 202 on the second side wall 232. The atomization surface 312 of the atomization core 30 cooperates with the accommodating cavity 21 to form an atomization cavity 211. An aerosol generated by the atomization surface 312 of the atomization core 30 heating the liquid substrate is released into the atomization cavity 211, and flows out of the atomizer 100 through the atomization cavity 211 for inhalation by the user.

Specifically, the atomization core 30 includes a porous substrate 31 with air permeability and a heating element 32 bonded to the porous substrate 31. The porous substrate 31 may be made of a hard capillarity structure, such as porous ceramic, porous glass-ceramic, or porous glass. The porous substrate 31 may be substantially, but is not limited to, a block structure in embodiments. According to the use situation, the liquid absorbing surface 311 and the atomization surface 312 are arranged opposite to each other on the porous substrate 31 in the thickness direction of the atomizer 100, that is, a direction perpendicular to the central axis L1 of the atomizer 100. The porous substrate 31 is mounted in the accommodating cavity 21 in an orientation such that the liquid absorbing surface 311 is parallel to the central axis L1. The liquid absorbing surface 311 is configured to absorb a liquid substrate, and the heating element 32 is bonded to the atomization surface 312 for heating and atomizing the liquid substrate. In some implementations, the liquid absorbing surface 311 and the atomization surface 312 of the atomization core 30 are each arranged parallel to the central axis L1 of the atomizer 100. In other implementations, the liquid absorbing surface 311 or the atomization surface 312 may alternatively be arranged obliquely to the central axis L1 of the atomizer 100.

In some implementations, the heating element 32 is made of materials, such as stainless steel, nickel-chromium alloy, iron-chromium-aluminum alloy, or titanium, and preferably, is formed on the atomization surface 312 by, after mixing a conductive raw material powder with printing aids to form a slurry, printing according to a suitable pattern and then sintering, so that all or most of the surface of the heating element 32 is tightly bonded to the atomization surface 312, which has effects such as high atomization efficiency, low heat loss, prevention of dry heating or significant reduction of dry heating. Optionally, the heating element 32 may be in various structural forms. For example, the heating element 32 may be a sheet-like heating element formed with a specific pattern bonded to the atomization surface 312, or in other forms such as a heating mesh, a disc-shaped heating element formed by spiraling a heating wire, and a heating film. For example, the specific pattern may be a serpentine shape.

Refer to FIG. 3, FIG. 4, and FIG. 5 to FIG. 10. The housing 10 is provided with an air outlet tube 102 inside, and the top wall 22 of the retaining seat 20 is provided with an air outlet hole 222. One end of the air outlet hole 222 is in communication with the atomization cavity 211, and the other end of the air outlet hole is in communication with the air outlet tube 102. Specifically, an end of the air outlet tube 102 is inserted into the air outlet hole 222, so that the air outlet tube 102 is in communication with the atomization cavity 211 through the air outlet hole 222. An air inlet cavity 45 is defined between the base 40 and the retaining seat 20. In a specific implementation, the mounting portion 41 and the annular flange 42 of the base 40 together with a bottom of the retaining seat 20 enclose to form the air inlet cavity 45. The air inlet cavity 45 is in communication with the atomization cavity 211. The base 40 is further provided with an air inlet column 46. In a specific implementation, a surface of the mounting portion 41 of the base 40 close to the retaining seat 20 is provided with an air inlet column 46. One end of the air inlet column 46 is in communication with the air inlet cavity 45, and the other end is in communication with the external atmosphere. The external gas enters the air inlet cavity 45 through the air inlet column 46, enters the atomization cavity 211 from the air inlet cavity 45, carries the aerosol in the atomization cavity 211 through the air outlet hole 222 to flow into the air outlet tube 102, and finally flows to the end opening of the air outlet tube 102 for inhalation by the user. In some implementations, an end of the ventilation groove 441 on the ventilation columns 44 away from the liquid storage cavity 101 may also extend into the air inlet cavity 45, so that the gas in the air inlet cavity 45 may enter the liquid storage cavity 101 through ventilation channels, for ventilating the liquid storage cavity 101.

In a specific implementation, the air inlet column 46 of the base 40, the atomization cavity 211 of the retaining seat 20, and the air outlet tube 102 are coaxially arranged. The projections of the atomization core 30 and the air inlet column 46 on a plane perpendicular to the central axis L1 of the atomizer 100 do not overlap with each other, so that the air flow channel from the air inlet column 46 to the air outlet hole 222 is a straight through channel, which can prevent the airflow entering through the air inlet column 46 from being blocked by the atomization core 30, and reduces dead zones of the airflow.

Refer to FIG. 3, FIG. 4, FIG. 9, and FIG. 10. The base 40 is further provided with two electrode mounting holes 47. The two electrode mounting holes 47 are arranged on the surface of the mounting portion 41 close to the retaining seat 20 and penetrate through the mounting portion 41. The two electrode mounting holes 47 are respectively arranged on two sides of the air inlet column 46. In a specific implementation, in the width direction of the atomizer 100, two electrode mounting holes 47 are respectively arranged on two sides of the air inlet column 46. The atomizer 100 further includes two conductive electrodes 50. The two conductive electrodes 50 penetrate through the two electrode mounting holes 47 in a one-to-one correspondence. Specifically, the conductive electrodes 50 are configured to be inserted into the atomization cavity 211 from the bottom of the retaining seat 20, and to abut against the atomization surface 312 of the atomization core 30.

In a specific implementation, the conductive electrodes 50 are arranged parallel to the central axis L1 of the atomizer 100, and the side surface of the conductive electrode 50 abuts against the heating element 32 on the atomization surface 312 of the atomization core 30. An end of the conductive electrode 50 away from the liquid storage cavity 101 is electrically connected to the battery core of the power supply assembly 200. The atomization core 30 achieves a stable electrical connection to the electric connection terminal of the power supply assembly 200 through the conductive electrode 50, so that the heating element 32 of the atomization core 30 can generate heat in a powered-on state, thereby heating and atomizing the liquid substrate to generate an aerosol. In other implementations, the conductive electrodes 50 may also be arranged obliquely to the central axis L1 of the atomizer 100, provided that the conductive electrodes 50 can achieve a stable electrical connection between the heating element 32 of the atomization core 30 and the electric connection terminal of the power supply assembly 200.

The foregoing descriptions are merely embodiments of this application, and are not intended to limit the patent scope of this application. All equivalent structures or equivalent process changes made according to the content of this specification and accompanying drawings of this application or direct or indirect application in other related technical fields are also included in the patent protection scope of this application.

## Claims

1. An atomizer, comprising:
a housing;
a retaining seat, arranged in the housing and cooperating with the housing to form a liquid storage cavity for storing a liquid substrate, wherein the retaining seat comprises a top wall and a side wall connected to each other; the top wall and the side wall enclose an accommodating cavity, and the top wall is provided with a ventilation hole; the side wall comprises a first side wall and a second side wall arranged opposite to each other, the first side wall is provided with a liquid inlet, the liquid inlet is in communication with the liquid storage cavity, and the second side wall is provided with an assembly hole;
a base, at least partially arranged in the housing and located at an end of the retaining seat away from the liquid storage cavity, wherein a ventilation column is arranged on the base, the ventilation column penetrates the ventilation hole, and the ventilation column cooperates with an inner surface of the ventilation hole to form a ventilation channel, one end of the ventilation channel is in communication with the liquid storage cavity, and the other end of the ventilation channel is in communication with an external atmosphere; and
an atomization core, arranged in the accommodating cavity, wherein the atomization core comprises a liquid absorbing surface for receiving the liquid substrate, and the liquid absorbing surface is communication with the liquid storage cavity through the liquid inlet, wherein
the assembly hole is configured to allow the atomization core to be mounted in the accommodating cavity through the assembly hole.

2. The atomizer according to claim 1, wherein the ventilation column is located on a side of the atomization core away from the liquid inlet and is spaced apart from the atomization core.

3. The atomizer according to claim 1, wherein an end portion of the side wall away from the liquid storage cavity is provided with an annular groove, and a bottom wall of the annular groove is provided with two arc-shaped grooves spaced apart from each other; and
the base comprises a mounting portion and an annular flange, and the annular flange is connected to a side of the mounting portion close to the retaining seat; two support portions spaced apart from each other are arranged on the annular flange in a direction away from the mounting portion; and the annular flange is embedded in the annular groove, and the two support portions are embedded in the two arc-shaped grooves in a one-to-one correspondence.

4. The atomizer according to claim 3, wherein the side wall further comprises a third side wall and a fourth side wall connected between the first side wall and the second side wall, the first side wall and the second side wall are arranged opposite to each other in a thickness direction of the atomizer, and the third side wall and the fourth side wall are arranged opposite to each other in a width direction of the atomizer; and
the two arc-shaped grooves are provided on the third side wall and the fourth side wall in a one-to-one correspondence.

5. The atomizer according to claim 1, wherein one end of the ventilation column is connected to the base, the ventilation column passes through the accommodating cavity, and the other end of the ventilation column extends into the liquid storage cavity, for the ventilation channel to come into communication with the accommodating cavity.

6. The atomizer according to claim 5, wherein a side surface of the ventilation column is provided with a ventilation groove, one end of the ventilation groove extends to an end surface of an end of the ventilation column away from the base, and the other end of the ventilation groove is in communication with the accommodating cavity.

7. The atomizer according to any one of claims 1 to 6, wherein the atomizer further comprises a fitting member, and the fitting member is mounted in the assembly hole; and/or
the atomization core further comprises an atomization surface, the atomization surface is arranged toward the assembly hole, the atomization surface cooperates with the accommodating cavity to form an atomization cavity; an air inlet cavity is defined between the base and the retaining seat, and the air inlet cavity is in communication with the atomization cavity; the base is provided with an air inlet column and two electrode mounting holes, and the two electrode mounting holes are respectively arranged on two sides of the air inlet column; and one end of the air inlet column is in communication with the air inlet cavity, and the other end of the air inlet column is in communication with the external atmosphere.

8. The atomizer according to claim 7, wherein the atomizer further comprises two conductive electrodes, and the conductive electrodes correspondingly penetrate the electrode mounting holes; and
the conductive electrodes are configured to be inserted from a bottom of the retaining seat into the atomization cavity and to abut against the atomization surface; and the ventilation column is configured to be inserted from the bottom of the retaining seat into the ventilation hole.

9. The atomizer according to claim 7, wherein the housing is provided with an air outlet tube inside, the top wall of the retaining seat is provided with an air outlet hole, one end of the air outlet hole is in communication with the atomization cavity, and the other end of the air outlet hole is in communication with the air outlet tube; and
the air inlet column, the atomization cavity, and the air outlet tube are coaxially arranged.

10. An electronic atomization device, comprising:
an atomizer, which is the atomizer according to any one of claims 1 to 9; and
a power supply assembly, electrically connected to the atomizer, configured to supply energy to the atomizer.
